# EUROPÄISCHE PATENTSCHRIFT

(11) **EP 2 276 450 B1**
(45) Veröffentlichungstag und Bekanntmachung des Hinweises auf die Patenterteilung: **21.08.2013**
(21) Anmeldenummer: 09731149.2
(22) Anmeldetag: 12.03.2009
(51) Int. Cl.: A61K 8/41, A61K 8/36, A61K 8/46, A61Q 17/04, A61Q 19/00

(54) **GETÖNTE ZUBEREITUNG FÜR DIE TAGESPFLEGE**
TINTED PREPARATION FOR DAY CARE
PRÉPARATION TEINTÉE POUR SOINS DE JOUR

(30) Priorität: 11.04.2008 DE 102008018786
(43) Veröffentlichungstag der Anmeldung: 26.01.2011
(73) Patentinhaber: Beiersdorf AG, 20253 Hamburg (DE)
(72) Erfinder: ESPEL, Anja, 22041 Hamburg (DE); BLOHM, Alexandra, 20257 Hamburg (DE); SCHÄFER, Jessica, 22113 Oststeinbek (DE); FEY, Sven, 22397 Hamburg (DE); RUPPERT, Stephan, 20259 Hamburg (DE)
(86) Internationale Anmeldenummer: PCT/EP2009/001787
(87) Internationale Veröffentlichungsnummer: WO 2009/124630

(56) Entgegenhaltungen:
- WO-A1-03/039502
- WO-A1-2004/022020
- WO-A1-2005/123013
- FLOESSER-MUELLER H ET AL: "Diethylamino Hydroxybenzoyl Hexyl Benzoate (DHHB) High Performance Protection against UV-induced Free Radicals in Skin" COSMETICS AND TOILETRIES MANUFACTURE WORLDWIDE, BUSHEY HEATH, GB, 1. Januar 2007 (2007-01-01), Seiten 31-37, XP009136490 ISSN: 1358-2453

## Beschreibung

Die vorliegende Erfindung betrifft eine kosmetische Zubereitung enthaltend 2-(4'-Diethylamino-2'-hydoxybenzoyl)-benzoesäurehexylester, ein oder mehrere Farbstoffe und Ölsäure.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren hat dabei der Zustand und das Aussehen der Haut.

Damit die Haut ihre biologischen Funktionen im vollen Umfang erfüllen kann, bedarf sie der regelmäßigen Reinigung und Pflege. Die Reinigung der Haut dient dabei der Entfernung von Schmutz, Schweiß und Resten abgestorbener Hautpartikel, die einen idealen Nährboden für Krankheitserreger und Parasiten aller Art bilden. Hautpflegeprodukte dienen meist der Befeuchtung und Rückfettung der Haut. Häufig sind ihnen Wirkstoffe zugesetzt, welche die Haut regenerieren oder vor den schädlichen Einflüssen der UV-Strahlung schützen.

Eine besondere Produktform von Hautpflegeprodukten stellen die Tagespflegeprodukte dar. Tagespflegeprodukte unterscheiden sich von den so genannten Nachtpflegeprodukten insbesondere darin, dass sie UV-Lichtschutzfilter enthalten (die man Nachts nicht benötigt), weniger stark fettend sind und schneller in die Haut einziehen. Tagespflegeprodukte können darüber hinaus mit Farbstoffen und Farbpigmenten versetzt sein, die der Haut nach dem Auftragen einen gesundes Aussehen verleihen. In der Regel enthalten sie Hautbefeuchtungsmittel (Moisturizer wie Glycerin oder Diole), kosmetische Wirkstoffe (beispielsweise gegen Falten) und andere Pflegestoffe (beispielsweise Ölkomponenten zur Rückfettung der Haut).

Ein wesentlicher Bestandteil moderner Tagespflegeprodukte sind die UVA-Lichtschutzfilter, die die Haut vor den schädlichen Einflüssen der UV-A-Strahlung schützen sollen. Ein in jüngerer Zeit entwickelter und für kosmetische Anwendungen zugelassener UV-A-Filter ist der 2-(4'-Diethylamino-2'-hydoxybenzoyl)-benzoesäurehexylester, welcher unter dem Handelsnamen Uvinul A Plus bei der Firma BASF erhältlich ist. Dieser UV-A- Filter hat gegenüber den herkömmlichen Filtern den Vorteil besonders UV-stabil und toxikologisch unbedenklich zu sein.

WO 2005/123013 A1 (BASF AG) offenbart die Verwendung von 2-(4'-Diethylamino-2'-hydoxybenzoyl)-benzoesäurehexylester zur Farbstabilisierung von kosmetischen und dermatologischen Zubereitungen.

Nachteilig an dieser Verbindung ist jedoch der Umstand, dass der Filter intensiv gelb gefärbt ist und kosmetische Zubereitungen, in die er eingearbeitet wird bei größerer Konzentration intensiv gelb färbt. Gelb gefärbte kosmetische Zubereitungen wie Cremes und Lotionen werden vom Verbraucher aber als unästhetisch empfunden. Bei der Anwendung auf der Haut kann es darüber hinaus passieren, dass die Haut durch den Farbton des Lichtschutzfilters selbst einen leichten und unangenehmen Gelbstich erhält. Statt einer gesunden sportlichen Röte oder Bräune bekommt die Haut eine ungesund (leberkrank) wirkende Farbe, was bei den Anwendern im Regelfall unerwünscht ist.

Nachteilig am Stande der Technik ist der Umstand, dass eine Reihe von Inhaltsstoffen von Tagespflegeprodukten beispielsweise aufgrund ihres Reinheitsgrades, ihrer Oxidationsempfindlichkeit oder schlicht ihrer Stoffeigenschaften, einen gewissen Eigengeruch aufweisen, der in der Regel als unangenehm und "unkosmetisch" empfunden wird. Das Problem des unerwünschten Eigengeruches tritt beispielsweise bei Ölsäure auf.

Derartige Eigengerüche von Inhaltsstoffen beeinträchtigen darüber hinaus das Duftbouquet der Parfümkomposition. Kurz: Derartige Eigengerüche von Inhaltsstoffen sind unerwünscht und müssen in der Regel mit einem übermäßigen Einsatz von teuren (und bei Einzelkomponenten manchmal auch dermatologisch nicht ganz unbedenklichen) Parfümstoffen überdeckt werden.

Es war daher die Aufgabe der vorliegenden Erfindung, die Nachteile de Standes der Technik zu beseitigen. Es war insbesondere die Aufgabe der vorliegenden Erfindung kosmetische Zubereitungen mit 2-(4'-Diethylamino-2'-hydoxybenzoyl)-benzoesäurehexylester zu entwickeln, die weniger intensiv gelb gefärbt sind und auf der Haut keinen "Gelbstich" hinterlassen.

Auch war es die Aufgabe der vorliegenden Erfindung Ölsäure-haltige Zubereitungen zu entwickeln, deren Ölsäure-Geruch deutlich und über einen längeren Zeitraum reduziert ist.

Überraschend gelöst werden die Aufgaben durch eine kosmetische Zubereitung enthaltend
a) 2-(4'-Diethylamino-2'-hydoxybenzoyl)-benzoesäurehexylester,
b) ein oder mehrere Farbstoffe in einer Gesamtmenge von 0,00001 bis 1 Gewichts-% bezogen auf das Gesamtgewicht der Zubereitung,
c) Ölsäure.

Die erfindungsgemäßen Zubereitungen lassen sich einfach und kostengünstig herstellen.

Es ist erfindungsgemäß vorteilhaft, wenn die Zubereitung ein oder mehrere Farbstoffe in einer Gesamtmenge von 0,00001 bis 1 Gewichts-%, bezogen auf das Gesamtgewicht der Zubereitung, enthält.

Erfindungsgemäß vorteilhafte Ausführungsformen der vorliegenden Erfindung sind dadurch gekennzeichnet, dass die Farbstoffe gewählt werden aus der Gruppe der Verbindungen mit dem Color Index CI 16035 (6-Hydroxy-5-(2-methoxy-5-methyl-4-sulfophenylazo)-2-naphthalinsulfonsäuredinatriumsalz, Alluarot) und Cl 15985 (1-(4-Sulfo-1-phenylazo)-2-naphthol-6-sulfosäure).

Enthält die erfindungsgemäße Zubereitung den Farbstoff Color Index CI 16035 (6-Hydroxy-5-(2-methoxy-5-methyl-4-sulfophenylazo)-2-naphthalinsulfonsäuredinatriumsalz, Alluarot), so ist es erfindungsgemäß vorteilhaft, diesen Farbstoff in einer Konzentration von 0,00001 bis 1 Gewichts-%, bezogen auf das Gesamtgewicht der Zubereitung, einzusetzen. Erfindungsgemäß bevorzugt ist eine Einsatzkonzentration von 0,00001 bis 0,2 Gewichts-%, bezogen auf das Gesamtgewicht der Zubereitung.

Enthält die erfindungsgemäße Zubereitung den Farbstoff Color Index CI 15985 (1-(4-Sulfo-1-phenylazo)-2-naphthol-6-sulfosäure), so ist es erfindungsgemäß vorteilhaft, diesen Farbstoff in einer Konzentration von 0,00001 bis 1 Gewichts-%, bezogen auf das Gesamtgewicht der Zubereitung, einzusetzen. Erfindungsgemäß bevorzugt ist eine Einsatzkonzentration von 0,00001 bis 0,2 Gewichts-%, bezogen auf das Gesamtgewicht der Zubereitung.

Es ist erfindungsgemäß vorteilhaft, wenn die Zubereitung 2-(4'-Diethylamino-2'-hydoxybenzoyl)-benzoesäurehexylester in einer Konzentration von 0,1 bis 12 Gewichts-%, bezogen auf das Gesamtgewicht der Zubereitung, enthält.

Es ist erfindungsgemäß bevorzugt, wenn die Zubereitung 2-(4'-Diethylamino-2'-hydoxybenzoyl)-benzoesäurehexylester in einer Konzentration von 0,5 bis 8 Gewichts-%, bezogen auf das Gesamtgewicht der Zubereitung, enthält.

Es ist erfindungsgemäß vorteilhaft, wenn die Zubereitung Ölsäure in einer Menge von 0,1 bis 10 Gewichts-%, bezogen auf das Gesamtgewicht der Zubereitung, enthält.

Es ist erfindungsgemäß bevorzugt, wenn die Zubereitung Ölsäure in einer Menge von 0,5 bis 5 Gewichts-%, bezogen auf das Gesamtgewicht der Zubereitung, enthält.

Es ist erfindungsgemäß vorteilhaft, wenn die erfindungsgemäße Zubereitung anorganische Farbpigmente, insbesondere Titandioxid, Eisenoxide und/oder Bariumsulfat enthält.

Vorteilhafte Ausführungsformen der vorliegenden Erfindung sind dadurch gekennzeichnet, dass die Zubereitung ein oder mehrere Diole in einer Gesamtkonzentration von 0,1 bis 8 Gewichts-%, bezogen auf das Gesamtgewicht der Zubereitung, enthält.

Erfindungsgemäß vorteilhafte Diole (ein oder mehrere) können erfindungsgemäß bevorzugt gewählt werden aus der Gruppe der Verbindungen 2-Methyl-1,3-propandiol, Propan-1,2-diol, Butan-1,2-diol, Pentan-1,2-diol, Hexan-1,2-diol, Heptan-1,2-diol, Octan-1,2-diol, Nonan-1,2-diol, Decan-1,2-diol.

Obwohl die erfindungsgemäße Zubereitung sowohl als wässrig-alkoholische Zubereitung, als Gel oder Oleogel vorliegen kann, ist es erfindungsgemäß vorteilhaft, wenn die Zubereitung in Form einer Emulsion oder Dispersion vorliegt. Erfindungsgemäß besonders bevorzugt sind dabei O/W-Emulsionen.

Liegt die erfindungsgemäße Zubereitung in Form einer O/W-Emulsion vor, so ist sie erfindungsgemäß bevorzugt dadurch gekennzeichnet, dass die Zubereitung einen oder mehrere O/W-Emulgatoren gewählt aus der Gruppe der Verbindungen Glycerylstearatcitrat, Glycerylstearat (selbstemulgierend), Stearinsäure, Stearatsalze, Polyglyceryl-3-methylglycosedistearat, Ceteareth-20, PEG-40 Stearat , Natriumcetearylsulfat, Sucrose Polystearat, Natrium Stearoyl Glutamat enthält. Ferner ist es vorteilhaft im Sinne der vorliegenden Erfindung Cetearylalkohol in Kombination mit PEG-40 hydriertes Rizinusöl, Natriumcetearylsulfat und Glycerylstearat. Ausserdem ist es erfindungsgemäß vorteilhaft Kaliumcetylphosphat als Emulgator einzusetzen.

Diese erfindungsgemäßen O/W-Emulgatoren können erfindungsgemäß vorteilhaft in einer Konzentration von 0,001 bis 10 Gewichts-% und bevorzugt in einer Konzentration von 0,1 bis 7 Gewichts-%, bezogen auf das Gesamtgewicht der Zubereitung in dieser enthalten sein.

In einer weiteren erfindungsgemäßen Ausführungsform liegt die erfindungsgemäße Zubereitung in Form einer W/O-Emulsion vor.

In dieser Ausführungsform ist es erfindungsgemäß bevorzugt, wenn die Zubereitung einen oder mehrere W/O-Emulgatoren gewählt aus der Gruppe der Verbindungen Polyglyceryl-2-dipolyhydroxystearat, PEG-30 Dipolyhydroxystearat, Cetyl Dimethicon Copolyol, Polyglyceryl-3 Diisostearat enthält.

Diese erfindungsgemäßen W/O-Emulgatoren können erfindungsgemäß vorteilhaft in einer Konzentration von 0,1 bis 10 Gewichts-% und bevorzugt in einer Konzentration von 0,2 bis 7 Gewichts-%, bezogen auf das Gesamtgewicht der Zubereitung in dieser enthalten sein.

Erfindungsgemäß vorteilhafte Ausführungsformen der vorliegenden Erfindung sind dadurch gekennzeichnet, dass die Zubereitung einen oder mehrere weitere UV-Filter enthält, gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 4-(tert.-Butyl)-4'-methoxydibenzoylmethan; Homomenthylsalicylat; 2-Ethylhexyl-2-hydroxybenzoat; 2-Ethylhexyl-2-cyano-3,3-diphenylacrylat; Dimethicodiethylbenzalmalonat; 3-(4-(2,2-bis Ethoxycarbonylvinyl)-phenoxy)propenyl)-methoxy-siloxan / Dimethylsiloxan - Copolymer; Dioctylbutylamidotriazon (INCl: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 2,4,6-Tris-(biphenyl)-1,3,5-triazin; 2,4-Bis-(4'- Di-neopentylaminobenzalmalonat)-6-(4"-butylaminobenzoat)-s-triazin , Titandioxid, Zinkoxid, Merocyanine gewählt aus der Gruppe der Verbindungen

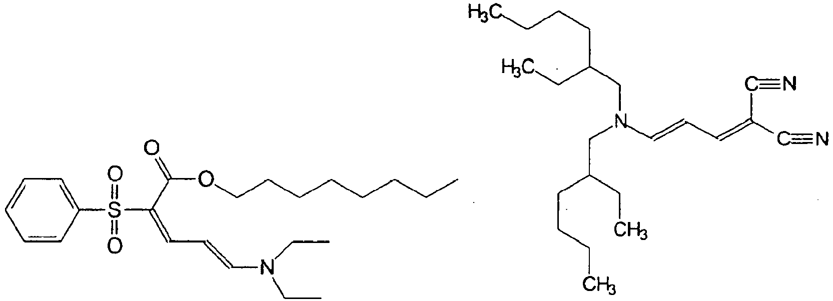

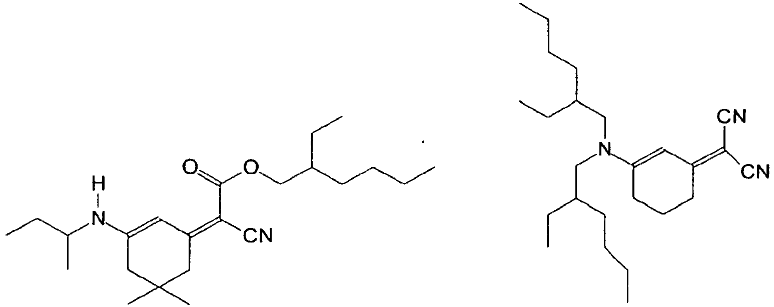

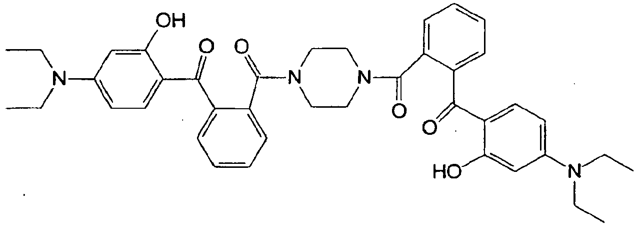

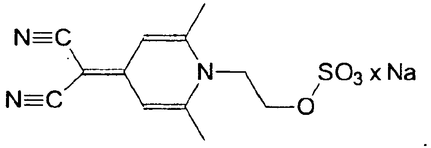

Es ist erfindungsgemäß vorteilhaft, wenn die erfindungsgemäße Zubereitung frei ist von p-Methylbenzylidencampher.

Es ist dabei erfindungsgemäß vorteilhaft, wenn die erfindungsgemäße Zubereitung einen oder mehrere dieser zusätzlichen UV-Filter in einer Gesamtkonzentration von 0,1 bis 40 Gewichts-% und bevorzugt in einer Konzentration von 1 bis 30 Gewichts-%, bezogen auf das Gesamtgewicht der Zubereitung, enthält.

Eine alternative erfindungsgemäße Ausführungsform der vorliegenden Erfindung ist dadurch gekennzeichnet, dass die Zubereitung keine UV-B-Filter enthält.

Es ist erfindungsgemäß vorteilhaft, wenn die Zubereitung als weitere Inhaltsstoffe eine oder mehrere Verbindungen gewählt aus der Gruppe der Verbindungen alpha-Liponsäure, Folsäure, Phytoen, D-Biotin, Coenzym Q10, Niacinamid, alpha-Glucosylrutin, Carnitin, Carnosin, natürliche und/oder synthetische Isoflavonoide, Flavonoide, Kreatin, Kreatinin, Taurin, ß-Alanin, Tocopherylacetat, Dihydroxyaceton; 8-Hexadecen-1,16-dicarbonsäure, Glycerylglycose, (2-Hydroxyethyl)harnstoff, Vitamin E bzw. seine Derivate und/oder Licochalcon A in den üblichen Einsatzkonzentrationen enthält.

Es ist erfindungsgemäß vorteilhaft, wenn die Zubereitung ein oder mehrere Parabene (z.B. Methylparaben, Ethylparaben, Propylparaben, Butylparaben) enthält.

Dabei ist ein Gesamt-Paraben-Gehalt von 0,05 bis 1 Gewichts-%, bezogen auf das Gesamtgewicht der Zubereitung erfindungsgemäß vorteilhaft.

Die erfindungsgemäßen Zubereitungen können ferner vorteilhaft auch Selbstbräunungssubstanzen enthalten, wie beispielsweise Dihydroxyaceton und/oder Melaninderivate in Konzentrationen von 1 Gew.-% bis zu 10 Gew.-%, bezogen auf das Gesamtgewicht der Zubereitung.

Ferner können die erfindungsgemäßen Zubereitungen auch Repellentien zum Schutz vor Mücken, Zecken und Spinnen und dergleichen enthalten. Vorteilhaft sind z. B. N,N-Diethyl-3-methylbenzamid (Handelsbezeichnung: Meta-delphene, "DEET"), Dimethylphtalat (Handelsbezeichnung: Palatinol M, DMP), 1-Piperidincarbonsäure-2-(2-hydroxyethyl)-1-methylpropylester sowie insbesondere 3-(N-n-Butyl-N-acetyl-amino)-propionsäureethylester (unter dem Handelsnamen Insekt Repellent® 3535 bei der Fa. Merck erhältlich). Die Repellentien können sowohl einzeln als auch in Kombination eingesetzt werden.

Als Feuchthaltemittel (Moisturizer) werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Weitere erfindungsgemäß vorteilhafte Feuchthaltemittel (Moisturizer) im Sinne der vorliegenden Erfindung (neben den Diolen) sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zum Schutz vor Hautveränderungen, wie sie z. B. bei der Hautalterung auftreten, verwendet werden.

Es ist vorteilhaft im Sinne der vorliegenden Erfindung, wenn die erfindungsgemäße Zubereitung ein oder mehrere der weiteren Feuchthaltemittel in einer Gesamtkonzentration von 0,1 bis 20 Gewichts-% und bevorzugt in einer Gesamtkonzentration von 0,5 bis 10 Gewichts-%, jeweils bezogen auf das Gesamtgewicht der Zubereitung, enthält.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9) und /oder Talkum.

Erfindungsgemäß vorteilhaft enthält die erfindungsgemäße Zubereitung Filmbildner. Filmbildner im Sinne der vorliegenden Erfindung sind Stoffe unterschiedlicher Zusammensetzung, die durch die folgende Eigenschaft charakterisiert sind: Löst man einen Filmbildner in Wasser oder anderen geeigneten Lösungsmitteln und trägt die Lösung dann auf die Haut auf, so bildet er nach dem Verdunsten des Lösemittels einen Film aus, der im wesentlichen dazu dient, die Lichtfilter auf der Haut zu fixieren und so die Wasserfestigkeit des Produktes zu steigern.

Es ist insbesondere von Vorteil, die Filmbildner aus der Gruppe der Polymere auf Basis von Polyvinylpyrrolidon (PVP) 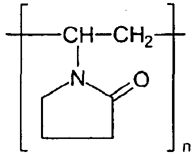 zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind.

Ebenfalls vorteilhaft sind weitere polymere Filmbildner, wie beispielsweise Natriumpolystryrensulfonat, welches unter der Handelsbezeichnung Flexan 130 bei der National Starch and Chemical Corp. erhältlich ist, und/oder Polyisobuten, erhältlich bei Rewo unter der Handelsbezeichnung Rewopal PIB1000. Weitere geeignete Polymere sind z.B. Polyacrylamide (Seppigel 305), Polyvinylalkohole, PVP, PVP / VA Copolymere, Polyglycole, Acrylat/Octylacralymid Copolymer (Dermacryl 79). Ebenfalls vorteilhaft ist die Verwendung von Hydriertem Rizinusöl Dimerdilinoleat (CAS 646054-62-8, INCI Hydrogenated Castor Oil Dimer Dilinoleate), das bei der Firma Kokyu Alcohol Kogyo unter dem Namen Risocast DA-H erworben werden kann oder aber auch PPG-3 Benzylethermyristat (CAS 403517-45-3), das unter dem Handelsnamen Crodamol STS bei der Firma Croda Chemicals erworben werden kann.

Die Ölphase der erfindungsgemäßen Zubereitung wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Jojobaöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl, Avocadoöl, Babassuöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Phenethylbenzoat, 2-Phenylethylbenzoat, Isopropyl Lauroyl Sarkosinat, Phenyl Trimethicon, Cyclomethicon, Dibutyladipat, Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isopropyl palmitat, Isostearyl Isostearat, Cetearyl Isononanoate, Isopropyl Stearat Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl) sowie Propylheptyl Octanoat und/oder Diisopropylsebacat.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol* CC bei der Fa. Cognis erhältliche.

Es ist ferner bevorzugt, das oder die Ölkomponenten aus der Gruppe Isoeikosan, Neopen-tylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Tridecylsalicylat (welches unter der Handelsbezeichnung Cosmacol ESI bei der Fa. Sasol erhältlich ist), C12-C15 Alkylsalicylat (unter der Handelsbezeichnung Dermol NS bei der Fa. Alzo erhältlich), Hexadecylbenzoat und Butyloctylbenzoat und Gemische davon (*Hallstar AB*) und/oder Diethylhexylnaphthalat (*Hallbrite TQ oder Corapan TQ von Symrise*).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene, C13-16 Isoparaffin und Isohexadecan. Unter den Polyolefinen sind Polydecene die bevorzugten Substanzen.

Es ist erfindungsgemäß besonders bevorzugt, wenn die Zubereitung Myristylmyristat enthält.

Die erfindungsgemäßen Zubereitungen können ferner vorteilhaft eine oder mehrere Substanzen aus der folgenden Gruppe der Siloxanelastomere enthalten, beispielsweise um die Wasserfestigkeit und/oder den Lichtschutzfaktor der Produkte zu steigern:
(a) Siloxanelastomere, welche die Einheiten R₂SiO und RSiO_{1,5} und/oder R₃SiO_{0,5} und/oder SiO₂ enthalten,
   wobei die einzelnen Reste R jeweils unabhängig voneinander Wasserstoff, C₁₋₂₄-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten R₂SiO zu RSiO_{1,5} aus dem Bereich von 1 : 1 bis 30 : 1 gewählt wird;
(b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind,
   wobei die verwendeten Mengenateile so gewählt werden, dass die Menge des Wasserstoffes des Organopolysiloxans (1) oder der ungesättigten aliphatischen Gruppen des Organopolysiloxans (2)
   - im Bereich von 1 bis 20 mol-% liegt, wenn das Organopolysiloxan nicht zyklisch ist und
   - im Bereich von 1 bis 50 mol-% liegt, wenn das Organopolysiloxan zyklisch ist.

Vorteilhaft im Sinne der vorliegenden Erfindung liegen das oder die Siloxanelastomere in Form sphärischer Puder oder in Form von Gelen vor.

Erfindungsgemäß vorteilhafte in Form sphärischer Puder vorliegende Siloxanelastomere sind die mit der INCI-Bezeichnung Dimethicone / Vinyl Dimethicone Crosspolymer, beispielsweise das von DOW CORNING unter der Handelsbezeichnungen DOW CORNING 9506 Powder erhältliche.

Besonders bevorzugt ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Die kosmetischen Zubereitungen gemäß der Erfindung können kosmetische Hilfsstoffe enthalten, wie sie üblicherweise in solchen Zubereitungen verwendet werden.

Vorteilhaft im Sinne der vorliegenden Erfindung sind Zubereitungen zur Pflege der Haut: sie können dem kosmetischen Lichtschutz, ferner als Schminkprodukt in der dekorativen Kosmetik dienen.

Erfindungsgemäß ist insbesondere die Verwendung der erfindungsgemäßen Zubereitung zum Schutz vor Hautalterung (insbesondere zum Schutz vor UV-bedingter Hautalterung) sowie als Sonnenschutzmittel.

Erfindungsgemäß vorteilhaft weist die erfindungsgemäße Zubereitung einen pH-Wert von 5 bis 8 auf. Dieser kann durch die herkömmlichen Säuren, Basen und Puffersysteme eingestellt werden.

Erfindungsgemäß ist die Verwendung der Zubereitung in kosmetischen Sprays (insbesondere Sonnenschutzmitteln) oder als Tränkmedium für Pads und Tücher.

### Vergleichsversuche

Mit den folgenden Vergleichsversuchen konnte der erfinderische Effekt gezeigt werden:

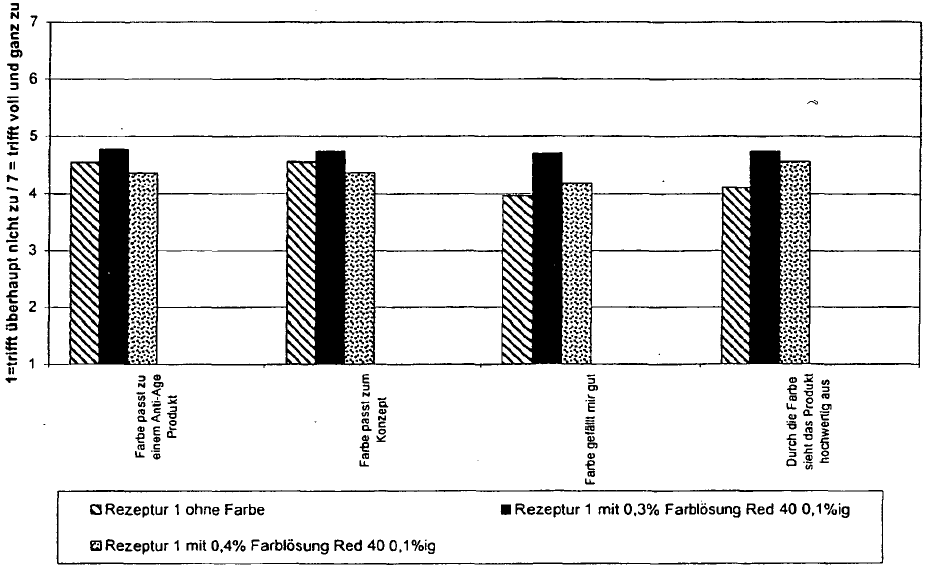

### Beschreibung des Testdesigns:

Bei der In-use Testung von verschiedensten Kosmetikprodukten werden pro Test max. 3 verschiedene Muster mit 25 Probanden getestet, bei dem pro Proband je ein Testmuster benötigt wird. Getestet wird die Akzeptanz des Produktes durch die Verbraucherinnen. Zusätzlich geschieht die Abfrage weiterer spezieller Eigenschaften, wie z.B. Hautgefühl, Pflegeeigenschaften. Die Testprodukte werden Verbraucher(innen), vornehmlich aus dem Haus BDF, mit nach Hause gegeben. Die Auswahl der Probanden kann zielgruppenspezifisch stattfinden: z.B. Verwenderinnen von Body Lotion, Frauen mit trockener Haut im Gesicht, etc.. In der Regel testen alle Probanden alle Testprodukte nacheinander, wobei die Reihenfolge von Proband zu Proband rotiert wird. Am Ende des Testzeitraums wird ein Fragebogen an die Auswerteabteilung zurückgesandt.

### Rezepturen des Vergleichsversuches:

| INCl | Rezeptur ohne Farblösung | mit 0,3% Farblösung | mit 0,4% Farblösung |
|---|---|---|---|
| Methylparaben | 0,10 | 0,10 | 0,10 |
| Caprylic/Capric Triglyceride | 1,00 | 1,00 | 1,00 |
| Aqua | 45,95 | 45,65 | 45,55 |
| Cera Microcristallina + Paraffinum Liquidum | 1,00 | 1,00 | 1,00 |
| Tocopheryl Acetate | 0,50 | 0,50 | 0,50 |
| Cetearyl Alcohol | 4,00 | 4,00 | 4,00 |
| Cetyl Alcohol | 3,00 | 3,00 | 3,00 |
| Glyceryl Stearate SE | 2,60 | 2,60 | 2,60 |
| Aqua + Trisodium EDTA | 1,00 | 1,00 | 1,00 |
| Phenoxyethanol | 0,40 | 0,40 | 0,40 |
| Dimethicone | 0,50 | 0,50 | 0,50 |
| C12-15 Alkyl Benzoate | 1,00 | 1,00 | 1,00 |
| Phenylbenzimidazole Sulfonic Acid | 1,50 | 1,50 | 1,50 |
| Glycerin | 8,70 | 8,70 | 8,70 |
| Aqua + Sodium Hydroxide | 0,65 | 0,65 | 0,65 |
| Ethylhexyl Salicylate | 3,75 | 3,75 | 3,75 |
| Butyrospermum Parkii Butter | 3,00 | 3,00 | 3,00 |
| Acrylates/C10-30 Alkyl Acrylate Crosspolymer | 0,10 | 0,10 | 0,10 |
| Ethylhexylgycerin | 0,50 | 0,50 | 0,50 |
| Biosaccharide Gum-1 | 5,00 | 5,00 | 5,00 |
| Chondrus Crispus | 0,20 | 0,20 | 0,20 |
| Butylene Glycol Dicaprvlate/Dicaprate | 3,50 | 3,50 | 3,50 |
| Methylpropanediol | 4,00 | 4,00 | 4,00 |
| Diethylamino Hydroxybenzoyl Hexyl Benzoate | 3,00 | 3,00 | 3,00 |
| Titanium Dioxide + Aluminum Hydroxide + Dimethicone/Methicone Copolymer | 0,75 | 0,75 | 0,75 |
| Parfüm | 0,30 | 0,30 | 0,30 |
| Aqua + Pimpinella Anisum Extract | 4,00 | 4,00 | 4,00 |
| **Farblösung Cl 16035 0,1%ig** | **0,00** | **0,30** | **0,40** |

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### O/W Emulsion

| | **1** | **2** | **3** | **4** | **5** | **6** |
|---|---|---|---|---|---|---|
| Glyceryl Stearat Citrat | 2 | 2 | 3 | | | |
| Glyceryl Stearat SE | | | | 1 | 1 | 1,5 |
| Cetearyl Alkohol + PEG-40 Rizinusöl+ Natrium Cetearyl Sulfat | | | | 2,5 | 2,5 | 3 |
| Cetearylalkohol | | | 1 | 1 | | |
| Stearylalkohol | 0,5 | | | | | 2 |
| Myristylmyristat | 1,0 | 1 | | | 3 | |
| Acrylates/C₁₀₋₃₀ Alkyl Acrylat Cross-polymer | 0,1 | 0,2 | | | 0,1 | |
| Carbomer | | 0,2 | 0,3 | 0,2 | | |
| Xanthan Gum | 0,4 | | 0,2 | 0,2 | 0,3 | 0,4 |
| C₁₂₋₁₅ Alkyl Benzoat | | 3 | | | 5 | |
| Butylenglycol Dicaprylat/Dicaprat | 5 | | | | 3 | 3 |
| Methylpropandiol | | 1 | | 0,5 | | |
| Dicaprylcaprat | 2 | 2 | | | 2 | 2 |
| 1,2-Hexandiol | 0,2 | | 0,1 | 0,3 | 0,1 | 0,1 |
| 1,2-Octandiol | | 0,2 | 0,1 | | 0,3 | |
| Cyclomethicon | | | 5 | 10 | | |
| PVP Hexadecen Copolymer | | 0,5 | | | | 1 |
| Propylenglykol | | | 1 | | 5 | 3 |
| Glycerin | 7,5 | 5 | 7 | 10 | 13 | 3 |
| Alkohol denat. | 2 | 3 | | 7 | | |
| Titandioxid | 3 | | | 2 | | |
| Ethylhexyltriazin | 2,5 | 2 | | 1 | | |
| 4-Methoxyzimtsäure(2-ethylhexyl)ester | | 5 | | 2 | | |
| Octocrylen | 7,5 | 9,5 | 3 | 2 | 2 | 1 |
| Butyl Methoxydibenzoylmethan | 3,8 | 4 | 3,5 | 2 | | |
| Phenylbenzimidazol Sulfonsäure | | | 1,5 | 2,5 | 3 | 2 |
| 2-(4'-Diethylamino-2'-hydoxybenzoyl)-benzoesäurehexylester | 1 | 2 | 5 | 3 | 7,5 | 10 |
| Ölsäure | 0,5 | 0,8 | 0,8 | 1 | 0,2 | 1,8 |
| Ethylhexylsalicylat | 2 | | 0,5 | 4 | 9,5 | |
| Bis-Ethylhexyloxyphenol Methoxy-phenyltriazin | | 1 | | 1 | | 1 |
| 2,2'-Methylen-bis-(6-(2H-benzotriazol-2-yl)-4-(1,1,3,3-tetramethylbutyl)-phenol) | | 4 | | | | 5 |
| 2,4,6-Tris-(biphenyl)-1,3,5-triazin | | | | 3 | 2 | |
| Vitamin E Acetat | 0,2 | 0,2 | 0,2 | 0,3 | 0,1 | 0,5 |
| Na₂H₂EDTA | 0,1 | 0,1 | 0,2 | 0,2 | 0,2 | 0,5 |
| Parfüm, Konservierungsmittel | q.s. | q.s. | q.s. | q.s. | q.s. | q.s. |
| Farbstoffe, usw. | q.s. | q.s. | q.s. | q.s. | q.s. | q.s. |
| Citronensäure, Natriumcitrat | q.s. | q.s. | q.s. | q.s. | q.s. | q.s. |
| Natriumhydroxid | q.s. | q.s. | q.s. | q.s. | q.s. | q.s. |
| Cl 15985 | 0,5 | 0,2 | 0,1 | | 0,5 | |
| Cl 16035 | | | | 0,2 | 0,01 | 0,01 |
| Wasser | ad 100,0 | ad 100,0 | ad 100,0 | ad 100,0 | ad 100,0 | ad 100,0 |

| | **7** | **8** | **9** | **10** | **11** | **12** |
|---|---|---|---|---|---|---|
| Glyceryl Stearat Citrat | 2 | 2 | 3 | | | |
| Glyceryl Stearat SE | | | | 1 | 1 | 1,5 |
| Cetearyl Alkohol | | | | 1,9 | 2,0 | 1,35 |
| PEG-40 Castoröl | | | | 0,375 | 0,4 | 0,225 |
| Natrium Cetearyl Sulfat | | | | 0,19 | 0,18 | 0,11 |
| Cetearylalkohol | | | 1 | 1 | | |
| Stearylalkohol | 0,5 | | | | | 2 |
| Myristylmyristat | 1,0 | 1 | | | 3 | 2 |
| Acrylat/C₁₀₋₃₀ Alkyl Acrylat Crosspolymer | 0,1 | 0,2 | | | 0,1 | |
| Carbomer | | 0,2 | 0,3 | 0,2 | | |
| Xanthan Gum | 0,4 | | 0,2 | 0,2 | 0,3 | 0,4 |
| C₁₂₋₁₅ Alkyl Benzoat | | 3 | | | 5 | |
| Butylenglycol Dicaprylat/Dicaprat | 5 | | | | 3 | 3 |
| Tridecylsalicylat | 1,5 | 2,5 | 0,25 | 5,9 | | |
| Dicaprylcaprat | 2 | 2 | | | 2 | 2 |
| Cyclomethicon | | | 5 | 10 | | |
| Dimethicon | | | | 5 | | |
| PVP Hexadecen Copolymer | | 0,5 | | | | 1 |
| Propylenglykol | | | 1 | | 5 | 3 |
| Glycerin | 7,5 | 5 | 7 | 10 | 13 | 3 |
| Alkohol denat. | 2 | 3 | | 7 | | |
| Titandioxid | 3 | | | 2 | | |
| Merocyanin | 2 | | 2 | | 3 | 3 |
| Ethylhexyltriazin | 2,5 | 2 | | 1 | | |
| 4-Methoxyzimtsäure(2-ethylhexyl)-ester | 9,5 | 5 | | 2 | | |
| Octocrylene | | 7,5 | 5 | 3 | | 1 |
| Butyl Methoxydibenzoylmethan | 3 | | | 2,7 | 4,5 | |
| 2-(4'-Diethylamino-2'-hydoxybenzoyl)-benzoesäurehexvlester | 1 | 2 | 5 | 1 | 2 | 7 |
| Phenylbenzimidazol Sulfonsäure | | | | 2,5 | 3 | |
| Methylpropandiol | 1 | 2 | | 3 | | 1 |
| Ölsäure | 0,5 | 1 | 0,8 | 2 | 4 | 0,1 |
| 1,2-Pentandiol | | 2 | 0,5 | 3 | 1 | |
| 1,2-Hexandiol | 0,2 | 0,1 | | 0,5 | 0,4 | |
| 1,2-Octandiol | | | 0,2 | | | 0,5 |
| Ethylhexylsalicylat | 2 | | 0,5 | 4 | 9,5 | |
| Bis-Ethylhexyloxyphenol Methoxy-phenyltriazin | | 1 | | 1 | | 1 |
| Vitamin E Acetat | 0,2 | 0,2 | 0,2 | 0,3 | 0,1 | 0,5 |
| Na₂H₂EDTA | 0,1 | 0,1 | 0,2 | 0,2 | 0,2 | 0,5 |
| Parfüm, Konservierungsmittel | q.s. | q.s. | q.s. | q.s. | q.s. | q.s. |
| Farbstoffe, usw. | q.s. | q.s. | q.s. | q.s. | q.s. | q.s. |
| Citronensäure, Natriumcitrat | q.s. | q.s. | q.s. | q.s. | q.s. | q.s. |
| Natriumhydroxid | q.s. | q.s. | q.s. | q.s. | q.s. | q.s. |
| Cl 15985 | 0,5 | 0,2 | 0,1 | | | |
| Cl 16035 | | | 0,5 | 0,2 | 0,01 | 0,01 |
| Wasser | ad 100,0 | ad 100,0 | ad 100,0 | ad 100,0 | ad 100,0 | ad 100,0 |

### Hydrodispersionen

| | **1** | **2** | **3** | **4** | **5** | **6** |
|---|---|---|---|---|---|---|
| Glyceryl Stearat Citrat | | 0,40 | | | | |
| Sodium Carbomer | | | | | 0,30 | |
| Acrylates/C10-30 Alkyl Acrylate Crosspolymer | | | 0,30 | 0,40 | 0,10 | 0,10 |
| Ceteareth-20 | | | 1,00 | | | |
| Xanthan Gummi | 0,50 | | | 0,15 | | 0,50 |
| Dimethicon / Vinyl Dimethicon Crosspolymer | | | | 5,00 | | 3,00 |
| 2-(4'-Diethylamino-2'-hydoxybenzoyl)-benzoesäurehexylester | 0,25 | 5,00 | 7,00 | 0,50 | 2,00 | 1,50 |
| Bis-Ethylhexyloxyphenol Methoxypheny Triazin | | 2,00 | | 0,25 | | |
| Ethylhexyl Methoxycinnamat | | | 7,00 | | 5,00 | 8,00 |
| Diethylhexyl Butamido Triazin | | | 2,00 | 1,00 | | |
| Ethylhexyl Triazin | 4,00 | 3,00 | | | 4,00 | |
| Titandioxid | 0,50 | 2,00 | 1,00 | 2,00 | 3,00 | 1,00 |
| Octocrylen | | 9,5 | 3 | 2 | | 7 |
| Butyl Methoxydibenzoylmethan | 4,2 | | 3,5 | | 4,5 | 1 |
| Methylpropandiol | 0,5 | 2 | 1,5 | 2,5 | 3 | 2 |
| C₁₂₋₁₅ Alkyl Benzoat | 2,00 | | 2,50 | | | |
| Olsäure | 0,5 | 1 | 2 | 0,5 | 5 | 1 |
| C18-36 Triglycerid Fettsäure | | | 1,00 | | | |
| Butylenglycol Dicaprylat/Dicaprat | 4,00 | | | | 6,00 | |
| Dicaprylyl Carbonat | | 3,00 | | | | |
| Dicaprylylether | | 2,00 | | | | |
| Cyclomethicon | | | | 7,50 | | |
| PVP Hexadecen Copolymer | 0,50 | | 0,50 | | 0,50 | 1,00 |
| Glycerin | 10,00 | 5,00 | 5,00 | | 5,00 | 15,00 |
| Butylenglycol | | 7,00 | | | | |
| 1,2-Hexandiol | 0,50 | | | 1,00 | | 1,50 |
| 1,2-Octandiol | | 0,4 | 0,2 | | 0,1 | |
| Vitamin E Acetat | 0,50 | 0,25 | 0,50 | 0,25 | 0,75 | 1,00 |
| Panthenol | 1,50 | 0,50 | | | 0,25 | |
| Trinatrium EDTA | | 1,00 | 1,00 | 0,10 | 0,20 | |
| Ethanol | 3,00 | | 4,00 | 3,50 | | 1,00 |
| Konservierungsmittel | q.s. | q.s. | q.s. | q.s. | q.s. | q.s. |
| Parfüm, Farbstoffe, | q.s. | q.s. | q.s. | q.s. | q.s. | q.s. |
| Cl 15985 | 0,5 | 0,2 | 0,1 | | | |
| Cl 16035 | | | 0,5 | 0,2 | 0,01 | 0,01 |
| Wasser | ad 100 | ad 100 | ad 100 | ad 100 | ad 100 | ad 100 |

### Gele

| | **1** | **2** | **3** | **4** | **5** | **6** |
|---|---|---|---|---|---|---|
| Acrylat/Octylacrylamid Copolymer | 1,0 | 1,0 | 1,0 | 1,0 | 1,0 | 1,0 |
| Alkohol Denat. | 50, 0 | 62,0 | 59,2 | 70,0 | 70,0 | 69,0 |
| Butylen Glycol Dicaprylat/Dicaprat | | | 9,5 | | | |
| C12-15 Alkyl Benzoat | 5,0 | 10,0 | 5,0 | 5,0 | 9,5 | |
| Phenylbenzoat | 5,0 | | | | | |
| Cocoglycerid | | | | | | 5 |
| Diethylamino Hydroxybenzoyl Hexyl Benzoat | 1,5 | 1 | 4,5 | 3,5 | 2,5 | 5 |
| Ethylhexyl Methoxycinnamat | 5 | 4,5 | | | 0,5 | |
| Ethylhexyl Salicylat | 4,5 | | 4,5 | 4,5 | | |
| Homosalat | | | | | | 4,5 |
| Hydroxypropylcellulose | 2 | 0,8 | 1 | 0,8 | 0,5 | 0,8 |
| Octocrylen | 7,5 | 9,5 | 3 | 2 | 2 | 3 |
| Butyl Methoxydibenzoylmethan | 4,2 | 4 | 3,5 | 2 | 4,5 | 3 |
| Methylpropandiol | 0,5 | 2 | 1,5 | 2,5 | 3 | 2 |
| 1,2-Hexandiol | | 0,3 | | 0,2 | | |
| 1,2-Octandiol | 0,4 | | 0,1 | 0,2 | 0,5 | 0,1 |
| Ethylhexyltriazin | | 2 | | | 2 | |
| Benzophenone-3 | 3 | | | | | |
| Bis-Ethylhexyloxyphenol Methoxy-phenyltriazin | | | 2,5 | | 3 | 1 |
| Ölsäure | 0,5 | 1 | 2 | 1,5 | 0,5 | 3 |
| Vitamin E Acetat | | | | 0,5 | | 0,2 |
| Glycerin | 5 | | 3 | | | |
| Parfüm, Farbstoffe | q.s. | q.s. | q.s. | q.s. | q.s. | q.s. |
| Cl 15985 | 0,5 | 0,2 | 0,1 | | | |
| Cl 16035 | | | 0,5 | 0,2 | 0,01 | 0,01 |
| Wasser | ad 100 | ad 100 | ad 100 | ad 100 | ad 100 | ad 100 |

| | | | |
|---|---|---|---|
| INCl | 1 | 2 | 3 |
| Methylparaben | - | 0,1 | 0,2 |
| Glyceryl Stearat | - | 3 | - |
| Caprylic/Capric Triglycerid | 1 | 1,5 | - |

| Wasser | ergänze zu 100 | ergänze zu 100 | ergänze zu 100 |
|---|---|---|---|
| Mikrokristallines Wachs | 0,7 | 1,5 | - |
| Paraffinöl | 0,5 | 1 | - |
| Vitamin E Acetat | 0,5 | 0,5 | - |
| Cetearyl Alkohol | 4 | 4 | 1,5 |
| Cetyl Alkohol | 3 | - | 1,5 |
| Glyceryl Stearat SE | 2,6 | 2,6 | - |
| Trisodium EDTA | 1 | 1 | 1 |
| BHT | - | - | 0,01 |
| Ethylhexyl Methoxycinnamat | - | - | 7,5 |
| Phenoxyethanol | - | 0,4 | 0,4 |
| Dimethicon | 0,5 | 0,5 | 2 |
| Hydrogenierte Coco-Glyceride | 2 | 2 - | 4 |
| C12-15 Alkyl Benzoat | 1 | 2 | - |
| Butyl Methoxydibenzoylmethan | - | 3 | 2 |
| Phenylbenzimidazol Sulfonsäure | 1,5 | 1,4 | - |
| Glycerin | 8,7 | 8,7 | 9,7 |

| Natronlauge | pH-Einstellung | pH-Einstellung | pH-Einstellung |
|---|---|---|---|
| Butylene Glycol | - | - | 3 |
| Ethylhexyl Salicylate | 3,75 | - | 4,5 |
| Karietebutter | 3 | 3 | 2,5 |
| Ölsäure | 0,5 | 1 | 0,2 |
| Acrylats/C10-30 Alkyl Acrylat Crosspolymer | 0,1 | 0,1 | 0,1 |
| Titan Dioxid + Trimethoxycaprylylsilan | - | 0,5 | - |
| Glyceryl Stearat Citrat | - | - | 2 |
| Cl 15985 | - | 0,7 | 0,5 |
| Cl 16035 | 0,5 | 0,2 | - |
| Ethylhexylglycerin | 0,25 | 0,5 | 0,25 |
| Octocrylene | - | 3 | - |
| Pentylen Glycol | 1 | - | - |
| Chondrus Crispus | 0.2 | 0,2 | - |
| Bis-Ethylhexyloxyphenol Methoxyphenyl Triazin | - | 1,75 | 0,5 |
| Butylen Glycol Dicaprylat/Dicaprat | 3,5 | - | - |
| Diethylamino Hydroxybenzoyl Hexyl Benzoate | 3 | 2 | 4,5 |
| Titan Dioxid + Aluminum Hydroxid + Dimethicon/Methicon Copolymer | 0,75 | - | 1 |
| Sodium Hyaluronat | 0,1 | 1 | 0,5 |
| Aqua + Pimpinella Anisum Extract | 5 | 2,5 | 4 |
| Parfum | 0,3 | 0,4 | 0,2 |
| Citronellol | 0,05 | 0,06 | 0,03 |

## Patentansprüche

1. Kosmetische Zubereitung enthaltend
a) 2-(4'-Diethylamino-2'-hydoxybenzoyl)-benzoesäurehexylester,
b) ein oder mehrere Farbstoffe in einer Gesamtmenge von 0,00001 bis 1 Gewichts-%, bezogen auf das Gesamtgewicht der Zubereitung, und
c) Ölsäure.

2. Kosmetische Zubereitung nach Anspruch 1, **dadurch gekennzeichnet, dass** die Farbstoffe gewählt werden aus der Gruppe der Verbindungen mit dem Color Index Cl 16035 und Cl 15985.

3. Kosmetische Zubereitung nach einem der vorhergehenden Ansprüche, **dadurch gekennzeichnet, dass** die Zubereitung 2-(4'-Diethylamino-2'-hydoxybenzoyl)-benzoesäurehexylester in einer Konzentration von 0,1 bis 10 Gewichts-%, bezogen auf das Gesamtgewicht der Zubereitung, enthält.

4. Kosmetische Zubereitung nach einem der vorhergehenden Ansprüche, **dadurch gekennzeichnet, dass** die Zubereitung Ölsäure in einer Menge von 0,1 bis 10 Gewichts-%, bezogen auf das Gesamtgewicht der Zubereitung, enthält.

5. Kosmetische Zubereitung nach einem der vorhergehenden Ansprüche, **dadurch gekennzeichnet, dass** die Zubereitung anorganische Farbpigmente, insbesondere Titandioxid, Eisenoxide und/oder Bariumsulfat enthält.

6. Kosmetische Zubereitung nach einem der vorhergehenden Ansprüche, **dadurch gekennzeichnet, dass** die Zubereitung ein oder mehrere Diole in einer Gesamtkonzentration von 0,1 bis 8 Gewichts-%, bezogen auf das Gesamtgewicht der Zubereitung, enthält.

7. Kosmetische Zubereitung nach einem der vorhergehenden Ansprüche, **dadurch gekennzeichnet, dass** die Zubereitung in Form einer Emulsion oder Dispersion vorliegt.

8. Kosmetische Zubereitung nach einem der vorhergehenden Ansprüche, **dadurch gekennzeichnet, dass** die Zubereitung einen oder mehrere weitere UV-Filter enthält, gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenme-thyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethyl-silyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäureamylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 4-(tert.-Butyl)-4'-methoxydibenzoylmethan; Homomenthylsalicylat; 2-Ethylhexyl-2-hydroxybenzoat; 2-Ethylhexyl-2-cyano-3,3-diphenylacrylat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCl: Diethylhexyl-Butamidotriazone); 2,4-bis-[5-1(dimethyl-propyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäure-tris-(2-ethylhexylester) (auch: 2,4,6-Tris-[anilino-(p-carbo-2'-ethyl-1'-hexyloxy)]-1,3,5-triazin (INCl: Ethylhexyl Triazone); 2,4-Bis-{[4-(2-ethyl-hexyloxy)-2-hydroxy]-phenyl}-6-(4-methoxyphenyl)-1,3,5-triazin (INCl: Bis-Ethylhexyloxyphenol Methoxyphenyl Triazin); 2,4,6-Tris-(biphenyl)-1,3,5-triazin; 2,4-Bis-(4'- Di-neopentylaminobenzalmalonat)-6-(4"-butylaminobenzoat)-s-triazin ; Titandioxid, Zinkoxid, Merocyanine gewählt aus der Gruppe der Verbindungen

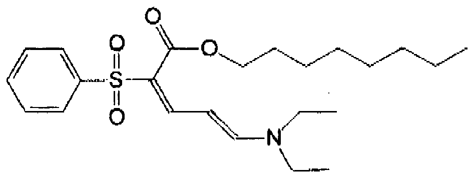

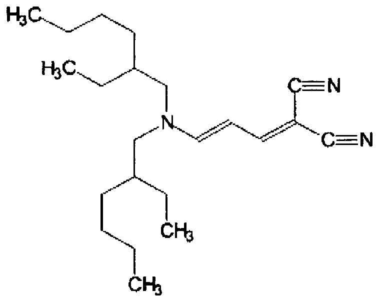

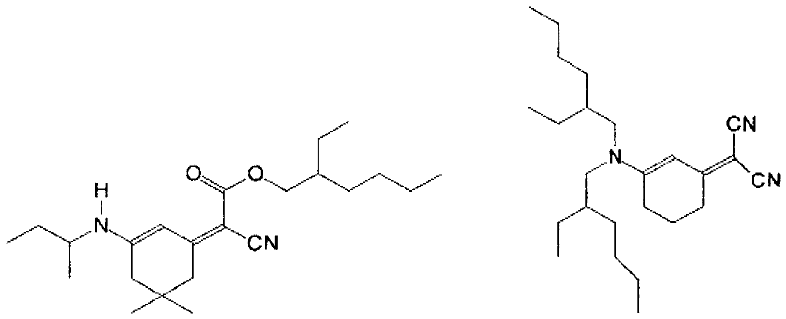

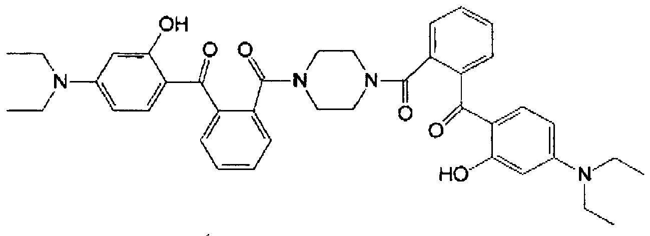

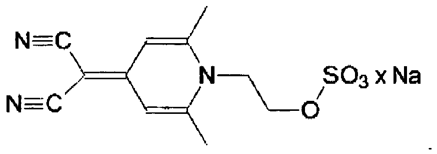

9. Kosmetische Zubereitung nach einem der vorhergehenden Ansprüche, **dadurch gekennzeichnet, dass** die Zubereitung als weitere Inhaltsstoffe eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, β-Alanin, Tocopherylacetat, Dihydroxyaceton; 8-Hexadecen-1,16-dicarbonsäure, Glycerylglycose, (2-Hydroxyethyl)harnstoff, Niacinamid ,Vitamin E bzw. seine Derivate und/oder Licochalcon A, enthält.

10. Kosmetische Zubereitung nach einem der Ansprüche 1 bis 7 und 9 **dadurch gekennzeichnet, dass** die Zubereitung keine UV-B-Filter enthält.

11. Kosmetische Zubereitung nach einem der vorhergehenden Ansprüche, **dadurch gekennzeichnet, dass** die Zubereitung Myristylmyristat enthält.

12. Kosmetische Zubereitung nach einem der vorhergehenden Ansprüche, **dadurch gekennzeichnet, dass** die Zubereitung ein oder mehrere Diole gewählt aus der Gruppe der Verbindungen 2-Methyl-1,3-propandiol, Pentan-1,2-diol, Hexan-1,2-diol, Heptan-1,2-diol, Octan-1,2-diol, Nonan-1,2-diol, Decan-1,2-diol enthält.

13. Kosmetische Zubereitung nach einem der vorhergehenden Ansprüche, **dadurch gekennzeichnet, dass** die Zubereitung ein oder mehrere Parabene enthält.

## Claims

1. Cosmetic preparation comprising
a) hexyl 2-(4'-diethylamino-2'-hydroxy-benzoyl)benzoate,
b) one or more dyes in a total amount of from 0.00001 to 1% by weight, based on the total weight of the preparation, and
c) oleic acid.

2. Cosmetic preparation according to Claim 1, **characterized in that** the dyes are selected from the group of the compounds with the colour index CI 16035 and CI 15985.

3. Cosmetic preparation according to one of the preceding claims, **characterized in that** the preparation comprises hexyl 2-(4'-diethylamino-2'-hydroxy-benzoyl)benzoate in a concentration of from 0.1 to 10% by weight, based on the total weight of the preparation.

4. Cosmetic preparation according to one of the preceding claims, **characterized in that** the preparation comprises oleic acid in an amount of from 0.1 to 10% by weight, based on the total weight of the preparation.

5. Cosmetic preparation according to one of the preceding claims, **characterized in that** the preparation comprises inorganic colour pigments, in particular titanium dioxide, iron oxides and/or barium sulphate.

6. Cosmetic preparation according to one of the preceding claims, **characterized in that** the preparation comprises one or more diols in a total concentration of from 0.1 to 8% by weight, based on the total weight of the preparation.

7. Cosmetic preparation according to one of the preceding claims, **characterized in that** the preparation is in the form of an emulsion or dispersion.

8. Cosmetic preparation according to one of the preceding claims, **characterized in that** the preparation comprises one or more further UV filters selected from the group of the compounds phenylene-1,4-bis(2-benzimidazyl)-3,3'-5,5'-tetrasulphonic acid salts; 2-phenylbenzimidazole-5-sulphonic acid salts; 1,4-di(2-oxo-10-sulpho-3-bornylidenemethyl)benzene and salts thereof; 4-(2-oxo-3-bornylidenemethyl)benzenesulphonic acid salts; 2-methyl-5-(2-oxo-3-bornylidenemethyl)sulphonic acid salts; 2,2'-methylene-bis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; ethylhexyl salicylate; terephthalidenedicamphorsulphonic acid; 2-ethylhexyl 4-(dimethylamino)benzoate; amyl 4-(dimethylamino)benzoate; di(2-ethylhexyl) 4-methoxy-benzalmalonate; 2-ethylhexyl 4-methoxycinnamate; isoamyl 4-methoxycinnamate; 2-hydroxy-4-methoxybenzophenone, 2-hydroxy-4-methoxy-4'-methylbenzophenone; 2,2'dihydroxy-4-methoxybenzophenone; 4-(tert-butyl)-4'-methoxy-dibenzoylmethane; homomenthyl salicylate; 2-ethylhexyl 2-hydroxybenzoate; 2-ethylhexyl 2-cyano-3,3-diphenyl-acrylate; dimethicodiethyl benzalmalonate; 3-(4-(2,2-bis(ethoxycarbonylvinyl)phenoxy)propenyl)methoxysiloxan e/dimethylsiloxane copolymer; dioctylbutylamidotriazone (INCI: Diethylhexyl Butamidotriazone); 2,4-bis[5-1(dimethylpropyl)benzoxazol-2-yl-(4-phenyl)imino]-6-(2-ethylhexyl)imino-1,3,5-triazine with the CAS No. 288254-16-0; tris(2-ethylhexyl) 4,4',4''-(1,3,5-triazine-2,4,6-triyltriimino)trisbenzoate (also: 2,4,6-tris[anilino(p-carbo-2'-ethyl-1'-hexyloxy)]-1,3,5-triazine (INCI: Ethylhexyl Triazone); 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine): 2,4,6-tris(biphenyl)-1,3,5-triazine; 2,4-bis(4'-dineopentylaminobenzalmalonate)-6-(4''-butylaminobenzoate)-s-triazine; titanium dioxide, zinc oxide, merocyanines selected from the group of the compounds

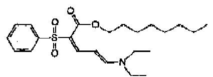

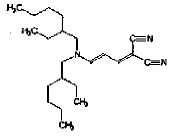

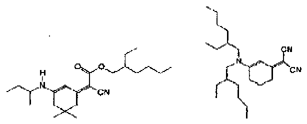

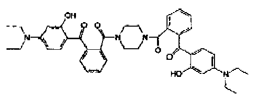

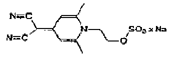

9. Cosmetic preparation according to one of the preceding claims, **characterized in that** the preparation comprises, as further ingredients, one or more compounds selected from the group of the compounds alpha-lipoic acid, folic acid, phytoene, D-biotin, coenzyme Q10, alpha-glucosylrutin, carnitine, carnosine, natural and/or synthetic isoflavonoids, flavonoids, creatine, creatinine, taurine, β-alanine, tocopheryl acetate, dihydroxyacetone; 8-hexadecene-1, 16-dicarboxylic acid, glyceryl glycose, (2-hydroxyethyl) urea, niacinamide, vitamin E and its derivatives and/or licochalcone A.

10. Cosmetic preparation according to one of Claims 1 to 7 and 9, **characterized in that** the preparation comprises no UV-B filters.

11. Cosmetic preparation according to one of the preceding claims, **characterized in that** the preparation comprises myristyl myristate.

12. Cosmetic preparation according to one of the preceding claims, **characterized in that** the preparation comprises one or more diols selected from the group of the compounds 2-methyl-1, 3-propanediol, pentane-1,2-diol, hexane-1,2-diol, heptane-1,7-diol, octane-1,2-diol, nonane-1,2-diol, decane-1,2-diol.

13. Cosmetic preparation according to one of the preceding claims, **characterized in that** the preparation comprises one or more parabens.

## Revendications

1. Préparation cosmétique contenant
a) de l'ester hexylique de l'acide 2-(4'-diéthylamino-2'-hydoxybenzoyl)-benzoïque,
b) un ou plusieurs colorants en une quantité totale de 0,00001 à 1% en poids par rapport au poids total de la préparation, et
c) de l'acide oléique.

2. Préparation cosmétique selon la revendication 1, **caractérisée en ce que** les colorants sont choisis dans le groupe des composés présentant le Color Index CI 16035 et CI 15985.

3. Préparation cosmétique selon l'une quelconque des revendications précédentes, **caractérisée en ce que** la préparation contient de l'ester hexylique de l'acide 2-(4'-diéthylamino-2'-hydoxybenzoyl)-benzoïque en une concentration de 0,1 à 10% en poids, par rapport au poids total de la préparation.

4. Préparation cosmétique selon l'une quelconque des revendications précédentes, **caractérisée en ce que** la préparation contient de l'acide oléique en une quantité de 0,1 à 10% en poids par rapport au poids total de la préparation.

5. Préparation cosmétique selon l'une quelconque des revendications précédentes, **caractérisée en ce que** la préparation contient des pigments colorés inorganiques, en particulier le dioxyde de titane, les oxydes de fer et/ou le sulfate de baryum.

6. Préparation cosmétique selon l'une quelconque des revendication précédentes, **caractérisée en ce que** la préparation contient un ou plusieurs diols en une concentration totale de 0,1 à 8% en poids, par rapport au poids total de la préparation.

7. Préparation cosmétique selon l'une quelconque des revendications précédentes, **caractérisée en ce que** la préparation se trouve sous forme d'une émulsion ou d'une dispersion.

8. Préparation cosmétique selon l'une quelconque des revendications précédentes, **caractérisée en ce que** la préparation contient un ou plusieurs autres filtres UV choisis dans le groupe des composés : sels de l'acide phénylène-1,4-bis-(2-benzimidazyl)-3,3'-5,5'-tétrasulfonique ; sels de l'acide 2-phénylbenzimidazole-5-sulfonique ; 1,4-di(2-oxo-10-sulfo-3-bornylidèneméthyl)-benzène et ses sels ; sels de l'acide 4-(2-oxo-3-bornylidèneméthyl)benzènesulfonique ; sels de l'acide 2-méthyl-5-(2-oxo-3-bornylidèneméthyl)sulfonique ; 2,2'-méthylène-bis-(6-(2H-benzotriazol-2-yl)-4-(1,1,3,3-tétraméthylbutyl)-phénol) ; 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; 3-(4-methylbenzylidène)camphre ; 3-benzylidènecamphre ; salicylate d'éthylhexyle ; acide téréphtalidènedicamphresulfonique ; ester 2-éthylhexylique de l'acide 4-(diméthylamino)-benzoïque ; ester amylique de l'acide 4-(diméthylamino)benzoïque ; ester di(2-éthylhexylique) de l'acide 4-méthoxybenzalmalonique ; ester 2-éthylhexylique de l'acide 4-méthoxycinnamique ; ester isoamylique de l'acide 4-méthoxycinnamique ; 2-hydroxy-4-méthoxybenzophénone, 2-hydroxy-4-méthoxy-4'-méthylbenzophénone ; 2,2'-dihydroxy-4-méthoxybenzophénone ; 4-(tert-butyl)-4'-méthoxydibenzoylméthane ; salicylate d'homomenthyle ; 2-hydroxybenzoate de 2-éthylhexyle ; 2-cyano-3,3-diphénylacrylatc de 2-éthylhexyle ; benzalmalonate de diméthicodiéthyle ; copolymère de 3-(4-(2,2-bis[éthoxycarbonylvinyl)-phénoxy)propényl)-méthoxysiloxane/diméthylsiloxane ; dioctylbutylamidotriazone (INCI : Diethylhexyl-Butamidotriazone) ; 2,4-bis-[5-1(diméthylpropyl)benzoxazol-2-yl-(4-phényl)-imino]-6-(2-éthylhexyl)-imino-1,3,5-triazine présentant le n° CAS 288254-16-0 ; ester tris(2-éthylhexylique) de l'acide 4,4',4"-(1,3,5-triazine-7,4,6-triyltriimino)-trisbenzoïque (également : 2,4,6-tris-[anilino-(p-carbo-2'-éthyl-1'-hexyloxy)]-1,3,5-triazine ; INCI : Ethylhexyl Triazone) ; 2,4-bis-{[4-(2-éthylhexyloxy)-2-hydroxy]-phényl}-6-(4-méthoxyphényl)-1,3,5-triazine (INCI : Bis-Ethylhexyloxyphenol Methoxyphenyl Triazin) ; 2,4,6-tris-(biphényl)-1,3,5-triazine ; 2,4-bis-(4'-dinéopentylaminobenzalmalonate)-6-(4"-butylaminobenzoate)-s-triazine ; dioxyde de titane, oxyde de zinc, mérocyanines choisies dans le groupe des composés

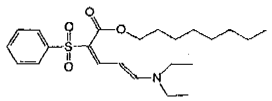

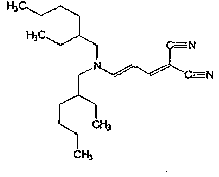

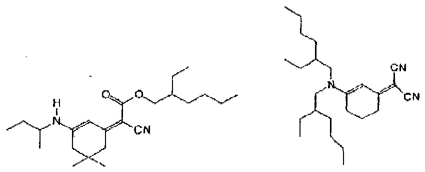

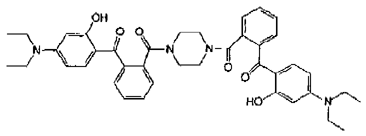

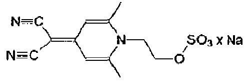

9. Préparation cosmétique selon l'une quelconque des revendications précédentes, **caractérisée en ce que** la préparation contient comme autres constituants un ou plusieurs composés choisis dans le groupe des composés acide alpha-liponique, acide folique, phytoène, D-biotine, coenzyme Q10, alpha-glucosylrutine, carnitine, carnosine, isoflavonoïdes naturels et/ou synthétiques, flavonoïdes, créatine, créatinine, taurine, β-alanine, acétate de tocophéryle, dihydroxyacétone ; acide 8-hexadécéne-1,16-diaarboxylique, glycérylglycose, (2-hydroxyéthyl)urée, niacinamide, vitamine E ou, selon le cas, ses dérivés et/ou licochalcone A.

10. Préparation cosmétique selon l'une quelconque des revendications 1 à 7 et 9, **caractérisée en ce que** la préparation ne contient pas de filtres UV-B.

11. Préparation cosmétique selon l'une quelconque des revendications précédentes, **caractérisée en ce que** la préparation contient du myristate de myristyle.

12. Préparation cosmétique selon l'une quelconque des revendications précédentes, **caractérisée en ce que** la préparation contient un ou plusieurs diols choisis dans le groupe des composés 2-méthyl-1,3-propanediol, pentane-1,2-diol, hexane-1,2-diol, heptane-1,2-diol, octane-1,2-diol, nonane-1,2-diol, décane-1,2-diol.

13. Préparation cosmétique selon l'une quelconque des revendications précédentes, **caractérisée en ce que** la préparation contient un ou plusieurs parabènes.
